(19) Europäisches Patentamt
European Patent Office
Office européen des brevets

(11) Numéro de publication: **0 165 853**
**B1**

(12) # FASCICULE DE BREVET EUROPÉEN

(45) Date de publication du fascicule du brevet: **12.08.87**

(51) Int. Cl.⁴: **C 07 C 149/16,** C 07 C 149/18, C 09 D 7/12, B 01 F 17/00

(21) Numéro de dépôt: **85401045.1**

(22) Date de dépôt: **28.05.85**

(54) **Nouveaux composés fluorés non ioniques, leur procédé d'obtention et leurs applications en tant que tensio-actifs.**

(30) Priorité: **29.05.84 FR 8408376**

(43) Date de publication de la demande: **27.12.85 Bulletin 85/52**

(45) Mention de la délivrance du brevet: **12.08.87 Bulletin 87/33**

(84) Etats contractants désignés: **AT BE CH DE FR GB IT LI LU NL SE**

(56) Documents cité:
**FR-A-2 111 253**
**FR-A-2 416 222**

(73) Titulaire: **INSTITUT NATIONAL DE RECHERCHE CHIMIQUE APPLIQUEE, 18 Bis, Boulevard de la Bastille, F-75012 Paris (FR)**

(72) Inventeur: **Lampin, Jean- Pierre, décédé (FR)**
Inventeur: **Cambon, Aimé, 59 Parc Mosca, F-0600 Nice (FR)**
Inventeur: **Szoni, François, 8 Bd d'Italie, Monaco Monte- Carlo (FR)**
Inventeur: **Delpuech, Jean- Jacques, 16 rue du Plateau, F-54520 Laxou (FR)**
Inventeur: **Serratrice, Guy, 20 rue Reherrey, F-54600 Villers- lès- Nancy (FR)**
Inventeur: **Thiollet, Gérard, 2 rue Gay Lussac, F-91610 Ballancourt (FR)**
Inventeur: **Lafosse, Louisette, 15 Allée des Lilas, F-91710 Vert- le- Petit (FR)**

(74) Mandataire: **Rinuy, Santarelli, 14, avenue de la Grande Armée, F-75017 Paris (FR)**

EP 0 165 853 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne de nouveaux composés répondant à la formule générale suivante:

$R_FC_2H_4S(R)H$,

où R désigne un motif $C_2H_4X$ (avec X = O ou S), ou une chaîne comprenant exclusivement plusieurs de tels motifs, deux motifs consécutifs pouvant être identiques ou différents, et

$R_F$ est un groupement alkyle perfluoré; l'invention couvre également des mélanges de tels composés, en particulier les mélanges des produits définis ci-dessus différant entre eux par la longueur de leur chaîne perfluorée, mais dont la partie hydrophile de la molécule est identique d'un produit à l'autre.

Les seuls produits connus de cette famille répondent aux formules:

$R_FC_2H_4SC_2H_4OH$,

$R_FC_2H_4S(C_2H_4O)_2H$ et

$R_F(C_2H_4S)_2H$

Les nouveaux composés de l'invention présentent des propriétés tensio-actives intéressantes.

Les tensio-actifs ont fait l'objet de nombreuses recherches depuis plusieurs années. Récemment, l'accent a été mis sur la nécessité de posséder des tensio-actifs définis afin d'obtenir des systèmes auto-émulsifiants stables composés de deux produits émulsifiants l'un plus hydrophile et l'autre plus hydrophobe (ou lipophile). On peut alors jouer sur la balance hydrophile/hydrophobe (HLB) de façon à obtenir une stabilité maximum pour les émulsions. De plus, pour certaines applications, il est nécessaire de disposer de tensio-actifs non ioniques.

Les composés et mélanges de l'invention répondent à ce besoin et constituent de nouveaux tensio-actifs non ioniques à partie hydrophile bien définie.

La mise au point de ces composés a été guidée par les deux critères suivants:

- nécessité de travailler sur des produits de départ disponibles industriellement;
- les produits préparés doivent avoir une partie hydrophile parfaitement définie et appartenir à des séries homogènes afin de permettre de régler divers paramètres tels que le HLB, le point de trouble (cloud point).

La présente invention vise ainsi un procédé d'obtention de ces composés et mélanges suivant une synthèse particulièrement souple donnant des produits comportant le nombre désiré et en particulier un nombre important de motifs d'oxyde d'éthylène.

Ainsi, selon l'invention, on fait réagir $R_FC_2H_4SH$ sur plusieurs monochlorhydrines d'éthylène- ou de polyéthylène- glycol définies selon le schéma suivant:

$$R_FC_2H_4SH \xrightarrow[Na]{EtOH} R_FC_2H_4SNa$$

$$\downarrow Cl(C_2H_4O)_nH$$

$$R_FC_2H_4S(C_2H_4O)_nH$$

avec en particulier $R_F = C_2F_5$, $C_4F_9$, $C_6F_{13}$, $C_8F_{17}$

avec en particulier n = (1,2,3,4)

pour obtenir un premier produit défini. On peut ainsi obtenir toute une gamme de produits définis avec des rendements de 70-80 %.

$R_FC_2H_4SH$ s'obtient lui-même facilement selon le schéma suivant:

$$R_FC_2H_4I + (NH_2)_2C{=}S \xrightarrow{\text{EtOH absolu}} R_FC_2H_4SC(NH_2) = \overset{\oplus}{N}H_2,\overset{\ominus}{I} \xrightarrow{\text{NaOH,aq.}} R_FC_2H_4SH$$

où $R_FC_2H_4I$ est un produit obtenu industriellement par fractionnement du produit de télomérisation du tétrafluoroéthylène et de l'iodure de perfluoroéthyle.

Pour augmenter le nombre de motifs éthers, on procède comme précédemment à l'aide d'un thiol correspondant, en remplaçant la fonction alcool du produit préalablement synthétisé par la fonction thiol que l'on fait réagir à nouveau sur des monochlorhydrines d'éthylène- ou de polyéthylène-glycol définies selon le schéma suivant:

$R_FC_2H_4S(C_2H_4O)_nH$ (A)

I ↓ a = HBr, $(NH_2)_2C = S$
ou
b = Tos Cl/pyridine, $(NH_2)_2C = S$ / NaOH

$R_RC_2H_4S(C_2H_4O)_{n-1}C_2H_4SH$ (B)

II ↓ EtO Na
$Cl(C_2H_4O)_{n'}H$ où n' = (1,2,3 ou 4)

$R_FC_2H_4S(C_2H_4O)_{n-1}C_2H_4S(C_2H_4O)_{n'}H$ (C)

pour obtenir un deuxième produit défini possédant deux motifs thio-éthers. On peut ainsi obtenir une deuxième gamme de produits définis.

Le même schéma pourra à son tour être appliqué au produit (C).

La voie Ia sera de préférence appliquée lorsque n = 1, la voie II de préférence lorsque n > 1.

Les produits obtenus sont des liquides, les plus longs des solides à bas point de fusion.

Ils peuvent être purifiés par distillation sous vide ou par simple filtration sur colonne de silice.

Si un procédé analogue est appliqué à un mélange de produits de la forme $R_FC_2H_4SH$ formé de thiols différant entre eux par la longueur de leur chaîne perfluorée $R_F$, on obtiendra un mélange de produits à partie hydrophile bien définie.

L'avantage essentiel du procédé en est donc sa souplesse. Du fait d'une part que le proton d'un thiol soit beaucoup plus mobile que celui d'un alcool et que d'autre part, les chlorhydrines à chaînes courtes ($n \leqslant 4$) soient commerciales, il s'ensuit qu'il est plus facile, pour obtenir un composé monodisperse à chaîne hydrophile donnée, d'opérer par ajouts successifs de motifs d'éthylène glycol. Les rendements sont ainsi plus élevés et comme, de plus, les premiers éléments de la famille sont aisément purifiables, les éléments comportant un grand nombre de motifs d'oxyde d'éthylène (en particulier plus de six motifs) deviennent aisément accessibles.

La présente invention concerne également les applications des produits et mélanges selon l'invention, en particulier en tant qu'agents tensio-actifs non ioniques: grâce à leurs propriétés tensio-actives, les composés selon l'invention peuvent être utilisés notamment comme additifs pour vernis thermodurcissables. Ils peuvent aussi être utilisés comme émulsionnants pour former des microémulsions, notamment avec des carbures fluorés ou perfluorés, par exemple avec la perfluorodécaline, les F-alkyléthènes, les perfluoroalkylamines (type "FC 43" ou "FTPA"), les bisperfluoroalkyléthènes, les di(F alkyl-2) thioéthanes ($R_FC_2H_4SC_2H_4R'_F$) et pour tout autre type d'émulsion ou microémulsion faisant intervenir des composés fluorés ou non à usage pharmacologique ou biologique.

Par microémulsion, on désigne des solutions optiquement isotropes limpides, et thermodynamiquement stables composées de fluorocarbure, d'eau et de tensio-actif en proportions variables, comme cela a été décrit dans le brevet N° 8 02 2875. Grâce à la possibilité d'ajuster à volonté la balance hydrophile/hydrophobe du tensio-actif, il a été constaté qu'il existe des zones de concentrations relatives de ces trois constituants pour lesquelles on obtient, à une température donnée, une microémulsion stable.

Les exemples suivants feront mieux ressortir la portée et l'intérêt de l'invention:

Exemple 1

$C_6F_{13}C_2H_4SC_2H_4OH$

Dans un réacteur de 2 litres balayé par un courant d'argon, on prépare l'éthylate à partir de 18,9 g de sodium (0,825 mole) dans 750 ml d'éthanol absolu. On introduit goutte à goutte 285 g de thiol $C_6F_{13}C_2H_4SH$ (0,75 mole). On obtient un liquide jaune clair. On refroidit alors avec un bain d'eau froide puis on ajoute goutte à goutte 0,75 mole de chloro-2-éthanol. Il se forme un précipité blanc de chlorure de sodium. On porte 3 heures au reflux pour terminer la réaction. On laisse refroidir, on hydrolyse avec 10 $cm^3$ d'eau permutée, on filtre pour séparer le chlorure de sodium, on sèche sur du sulfate de magnésium, on filtre, on chasse le solvant puis on distille.

On obtient un produit présentant un P.E. = 130-133°C/18 mm Hg avec un rendement de 72 %.

Le produit a été analysé par C.P.G. et RMN du proton.

pureté par chromatographie: 98,6 %

RMN du proton :

| Motif | $\delta$ (ppm) /TMS | Intensité |
|---|---|---|
| $CH_2O$ | 3,8 | 2 |
| $CH_2S$ | 2,77 | 4 |
| $CH_2$ | 2,3 | 2 |

De façon analogue, les produits suivants ont été préparés: $R_FC_2H_4S(C_2H_4O)_nH$ en utilisant différents thiols $R_FC_2H_4SH$ et différentes chlorhydrines $Cl(C_2H_4O)_nH$.

| $R_F$ | n | P.E. °C/mm Hg | Rendement % | Pureté par C.P.G. % |
|---|---|---|---|---|
| $C_6F_{13}$ | 1 | 130-133 / 18 | 72 | 98,6 |
| $C_6F_{13}$ | 2 | 118-120 / 1,3 | 76,5 | 98,5 |
| $C_6F_{13}$ | 3 | 137-139 / 0,7 | 86 | 95,2 |
| $C_6F_{13}$ | 4 | 173-175 /10,9 | 69,5 | 89,8 |
| $C_2F_5$ | 1 | 85-90 /20 | 80 | 97,8 |
| $C_2F_5$ | 2 | 80-85 / 1,4 | 82 | 98,5 |
| $C_2F_5$ | 3 | 110 / 1,4 | 83 | 96,8 |
| $C_4F_9$ | 1 | 70 / 1,3 | 86,5 | 98,6 |
| $C_4F_9$ | 2 | 105-110 / 1,45 | 79 | 98,5 |
| $C_4F_9$ | 3 | 125 / 1,2 | 75 | 97,9 |

Exemple 2

$C_6F_{13}C_2H_4SC_2H_4SH$

Dans un Erlenmeyer rodé de 1 litre, balayé par un courant d'argon, équipé d'un réfrigérant et d'une agitation magnétique, on place 0,05 mole d'alcool $C_6F_{13}C_2H_4SC_2H_4OH$, 0,05 mole de thiourée et 0,15 mole de HBr à 48 %. On chauffe l'ensemble à 100°C pendant 20 heures sous agitation. Il se forme un précipité qui se colle sur les parois. On laisse refroidir puis on ajoute une solution de soude préparée à partir de 0,15 mole dans 600 ml d'eau puis on chauffe encore deux heures à reflux.

On refroidit avec un bain d'eau glacée, puis on acidifie avec de l'acide chlorhydrique N. On extrait au chlorure de méthylène, on lave la couche orginique à l'eau, décante, sèche sur du sulfate de magnésium puis on chasse le solvant et on distille.

On obtient un produit présentant un P.E. de 125-127°C/18 mm Hg avec un rendement de 64 %.

Pureté par chromatographie: 94,9 %

Exemple 3

$C_6F_{13}C_2H_4SC_2H_4OC_2H_4SH$

1. Synthèse du tosylate

Dans un réacteur de 2 litres équipé d'un agitateur, d'un réfrigérant surmonté d'une garde à chlorure de calcium, on place 0,25 mole d'alcool $C_6F_{13}C_2H_4SC_2H_4OC_2H_4OH$ et 1 mole de pyridine. On refroidit à 10°C et on ajoute, au moyen d'une ampoule à solide, par fraction, 0,3 mole de chlorure de p-toluène sulfonyle en 30 minutes environ, de façon à maintenir la température inférieure à 20° C.

Le mélange est alors agité 4 heures à cette température, puis on ajoute une solution d'acide chlorhydrique préparée à partir de 150 ml d'acide 12N dans 1,5 l d'eau.

On extrait au chlorure de méthylène, décante, sèche la phase organique sur du sulfate de magnésium, filtre puis on chasse le solvant. Le tosylate obtenu est un liquide visqueux que l'on utilise tel quel pour la suite de la synthèse.

Rendement: 99 %

2. Passage au thiol

Dans un réacteur de 1 litre équipé d'un réfrigérant, d'une agitation, d'une ampoule à brome et balayé par un courant d'argon, on introduit le tosylate précédent (0,247 mole), 0,37 mole de thiourée et 400 ml d'éthanol

absolu. Le mélange est chauffé au reflux de l'éthanol pendant 20 heures. On laisse refroidir puis on chasse l'éthanol.

Dans un réacteur de 1 litre inerte, on place le résidu précédent et on ajoute 200 ml d'eau puis on chauffe sous agitation à 70° C. A cette température, on ajoute 13,8 g de soude dans 200 ml d'eau; on maintient pendant 2 heures à cette température. On refroidit, acidifie le milieu avec HCl N, extrait au chlorure de méthylène, sèche la couche organique, chasse le solvant puis on distille.

On obtient un produit avant un P.E. = 155-156° C/18 mm Hg avec un rendement de 59 %.

Le produit est analysé par chromatographie et RMN du proton.

Pureté par chromatographie: 96 %

RMN du proton :

| Motif | $\delta$ (ppm)/TMS | Intensité |
|---|---|---|
| $CH_2O$ | 3,61 | 4 |
| $CH_2S$ | 2,76 | 6 |
| $CH_2$ | 2,5 | 2 |
| SH | 1,58 | 1 |

On prépare de la même façon $C_6F_{13}C_2H_4S(C_2H_4O)_2C_2H_4SH$ à partir de $C_6F_{13}C_2H_4S(C_2H_4O)_3H$.

P.E. = 124-125° C/0,5 mm Hg avec un rendement de 86 %. Pureté par chromatographie: 93,4%

RMN du proton :

| Motif | ppm/TMS | Intensité |
|---|---|---|
| $CH_2O$ | 3,63 | 8 |
| $CH_2S$ | 2,76 | 6 |
| $CH_2$ | 2,51 | 2 |
| SH | 1,58 | 1 |

Exemple 4

$C_6F_{13}C_2H_4SC_2H_4S(C_2H_4O)_3H$

Dans un réacteur de 250 ml inerté, on prépare un éthylate à partir de 0,077 mole de sodium dans 100 ml d'éthanol absolu. Lorsque tout le sodium est attaqué, on ajoute 0,07 mole de thiol $C_6F_{13}C_2H_4SC_2H_4SH$ pendant 15 minutes. Il n'y a pas d'élévation de température pendant la coulée. On agite 15 minutes après la fin de la coulée puis on ajoute 0,07 mole de chlorhydrine pendant 15 minutes. On chauffe alors pendant trois heures au reflux de l'éthanol. On refroidit puis on hydrolyse avec 20 cc d'eau permutée pour éliminer l'excès d'éthylate de sodium. On extrait au chlorure de méthylène, sèche la couche organique, filtre et chasse le solvant ayant de distiller.

On obtient un produit ayant un P.E. de 160-162° C/0,2 mm Hg avec un rendement de 43 %.

Pureté par chromatographie: 98,5%.

RMN du proton :

| Motif | $\delta$ (ppm)/TMS | Intensité |
|---|---|---|
| $CH_2O$ | 3,65 | 10 |
| $CH_2S$, $CH_2$ | 2,80 | 10 |

On prépare de la même façon $C_6F_{13}C_2H_4SC_2H_4OC_2H_4S(C_2H_4O)_2H$ à partir de $C_6F_{13}C_2H_4SC_2H_4OC_2H_4SH$ et $Cl(C_2H_4O)_2H$.

P.E. = 157-158° C/0,12 mm Hg avec un rendement de 72%.

Pureté par chromatographie: 97,5 %.

RMN du proton :

| Motif | δ ppm/TMS | Intensité |
|---|---|---|
| $CH_2O$ | 3,68 | 10 |
| $CH_2S$ | 2,75 | 8 |
| $CH_2$ | 2,38 | 2 |
| OH | 2,53 | 1 |

De façon analogue, on prépare $C_6F_{13}C_2H_4S(C_2H_4O)_2C_2H_4SC_2H_4OH$ à partir de $C_6F_{13}C_2H_4S(C_2H_4O)_2C_2H_4SH$ et de $ClC_2H_4OH$.

P.E. = 165-170° C/10,6 mm Hg avec un rendement de 78 %.

Pureté par chromatographie: 99,7 %.

RMN du proton :

| Motif | δ ppm/TMS | Intensité |
|---|---|---|
| $CH_2O$ | 3,63 | 10 |
| $CH_2S$ | 2,72 | 8 |
| $CH_2$ | 2,35 | 2 |

En procédant de la même manière, à partir de $C_6F_{13}C_2H_4SC_2H_4OC_2H_4SH$ et de $Cl(C_2H_4O)_3H$, on prépare $C_6F_{13}C_2H_4S(C_2H_4O)_3H$

P.E. = 191-193° C/0,3 mm Hg.

Pureté par chromatographie: 99 %.

Exemple 5

Mélanges de produits de la forme $R_FC_2H_4S(C_2H_4O)_3H$

On opère comme dans l'exemple 1 en remplacant le thiol $C_6F_{13}C_2H_4SH$ par une coupe industrielle correspondant à un mélange A de thiols dans les proportions suivantes (analyse en C.P.G.):

30,5 % de $C_6F_{13}C_2H_4SH$
34,3 % de $C_8F_{17}C_2H_4SH$
27,3 % de $C_{10}F_{21}C_2H_4SH$
4,2 % de $C_{12}F_{25}C_2H_4SH$

Dans un réacteur de 500 ml balayé par un courant d'argon, on prépare l'éthylate à partir de 5,06 g (0,22 mole) dans 200 ml d'éthanol absolu. Lorsque tout le sodium est attaqué, on abaisse la température que l'on maintient au bain d'eau à température ambiante et on ajoute 96 g du mélange de thiols A. On agite 15 minutes et on introduit 33,7 g de chlorhydrine (0,2 mole). On chauffe 3 heures au reflux de l'éthanol.

Le mélange est brun coloré et NaCl précipite. On hydrolyse avec 10 cc d'eau permutée, on filtre le chlorure de sodium et on chasse l'éthanol.

On reprend le résidu au chloroforme, on lave à l'eau et on sèche sur du sulfate de magnésium. On filtre puis on chasse le solvant. Le résidu très coloré est distillé sous 0,2 mm Hg. On recueille (P.E/0,2 mm Hg = 132-167° C) 122,4 g d'un produit ayant la consistance d'une cire dont l'analyse par C.P.G. révèle qu'il contient :

33,8 % de $C_6F_{13}C_2H_4S(C_2H_4O)_3H$
35,4 % de $C_8F_{17}C_2H_4S(C_2H_4O)_3H$
24,8 % de $C_{10}F_{21}C_2H_4S(C_2H_4O)_3H$
3,1 % de $C_{12}F_{25}C_2H_4S(C_2H_4O)_3H$

II. Exemples d'application

1. Comme additifs tensio-actifs pour vernis thermodurcissables

Essai N° 1

On ajoute 2 parts de $C_6F_{13}C_2H_4S(C_2H_4O)_3H$ à 1000 parts de vernis thermodurcissable "IRDALON 300.1" préparé selon l'exemple 1 de la demande de brevet français publiée sous le N° 2.523.590.

La composition du revêtement ainsi obtenu est étalée sur une plaque de polycarbonate (LEXAN fabriqué par General Electric) de 100 X 100 X 2 (mm) par immersion (vitesse d'immersion 120 mm/minute), puis durcie par chauffage à 120° C pendant 75 minutes.

La plaque revêtue est ensuite soumise au test d'abrasion ASTM D 1044 TABER au moyen d'un abrasimètre TABER.

Ce test consiste à mesurer le pourcentage de trouble optique au moyen d'un "Hazemeter" après passage de l'échantillon sous deux meules abrasives lestées pendant un nombre de tours déterminé.

Pour cet exemple, la charge sur les meules abrasives était de 1000 g et le nombre de tours égal à 100.

Le pourcentage de trouble mesuré a été de 1,1 % contre 3,5 % pour une plaque témoin revêtue d'un vernis ne contenant pas cet agent tensio-actif et 30 % pour une plaque non revêtue.

Essai N° 2

On ajoute 2 parts de $C_6F_{13}C_2H_4SC_2H_4OC_2H_4S(C_2H_4O)_2H$ à 1000 parts de vernis thermodurcissable "IRDALON 300.1"

On opère ensuite de la même façon que dans l'essai N° 1. Le pourcentage de trouble a été ici de 0,9 %.

2. Pour les microémulsions

Le présent exemple montre la possibilité d'obtention de microémulsions de fluorocarbures en utilisant un tensio-actif fluoré non ionique selon l'invention.

On a en effet trouvé qu'il existe des zones de concentrations relatives des trois constituants: eau, fluorocarbure, agent tensio-actifs, pour lesquelles on obtient à une température donnée, une microémulsion stable. Il importe alors de déterminer pour un fluorocarbure donné, le diagramme termaire comportant les zones de concentrations qui procurent une microémulsion l'impide et stable à la température souhaitée. Ces zones peuvent être de deux types: celles où le constituant le plus abondant en volume est l'eau (type huile dans l'eau) et celles où le constituant le plus abondant en volume est le fluorocarbure (type eau dans huile).

Les exemples de mélanges ternaires ci-dessous montrent la versatilité des systèmes vis-à-vis de la température et leur intérêt éventuel pour des applications biologiques.

Microémulsions du type "huile dans l'eau"

Exemple 1

Préparation d'une microémulsion

$H_2O/C_8F_{17}CH=CH_2/C_6F_{13}C_2H_4S(C_2H_4O)_3H$ stable à 25°C.

Dans un tube à essais, on a ajouté 1,0 g de $C_6F_{13}C_2H_4S(C_2H_40)_3H$, 7,1 g de $H_2O$ et 1,9 g de $C_8F_{17}CH=CH_2$.

Après agitation mécanique pendant quelques minutes et un bref chauffage à une température supérieure à 25°C, le mélange a été placé dans un bain thermostat à 25°C; on a obtenu une solution limpide et stable à cette température.

Exemple 2

Préparation d'une microémulsion

$H_2O/C_8F_{17}CH=CH_2/C_6F_{13}C_2H_4S(C_2H_4O)_3H$ stable à 37° C.

Dans un tube à essais, on a ajouté 1,5 g de $C_6F_{13}C_2H_4S(C_2H_4O)_3H$, 6,0 g de $H_2O$ et 2,5 g de $C_8F_{17}CH=CH_2$.

Après agitation mécanique pendant quelques minutes et un bref chauffage à une température supérieure à 37°C, le mélange a été placé dans un bain thermostaté à 37°C; on a obtenu une solution limpide et stable à cette température.

Exemple 3

Préparation d'une microémulsion

$H_2O/C_8F_{17}CH=CH_2/C_6F_{13}(C_2H_4S)_2(C_2H_4O)_3H$ stable à 25°C.

Dans un tube à essais, on a ajouté 1,5 g de $C_6F_{13}(C_2H_4S)_2(C_2H_4O)_3H$, 4,1 g de $H_2O$ et 4,4 g de $C_8F_{17}CH=CH_2$.

Après agitation mécanique et un chauffage à une température supérieure à 25° C pendant quelques minutes, le mélange a été placé dans un bain thermostaté à 25° C; on a obtenu une solution limpide et stable à cette température.

Exemple 4

Préparation d'une microémulsion

$H_2O$/perfluorodécaline/$C_6F_{13}C_2H_4S(C_2H_4O)_3H$ stable à 30°C.

Dans un tube à essais, on a ajouté 1,01 g de $C_6F_{13}C_2H_4S(C_2H_4O)_3H$ 5,58 g de $H_2O$ et 3,41 g de perfluorodécaline.

Aprés agitation mécanique et un chauffage à une température supérieure à 30°C pendant quelques minutes, le mélange a été placé dans un bain thermostaté à 30° C; on o abtenu une solution limpide et stable à cette température.

Exemple 5

Préparation d'une microémulsion

$H_2O/C_4F_9CH=CHC_4F_9/C_6F_{13}C_2H_4S(C_2H_4O)_3H$ stable à 20° C.

Dans un tube à essais, on a ajouté 1,53 g de $C_6F_{13}C_2H_4S(C_2H_4O)_3H$, 5,95 g de $H_2O$ et 2,52 g de $C_4F_9CH=CHC_4F_9$.

On a opéré selon le mode opératoire défini précédemment et on a obtenu une microémulsion stable à 20° C.

Exemple 6

Préparation d'une microémulsion

$H_2OC_8F_{17}CH = CH_2/C_6F_{13}C_2H_4S(C_2H_4O)_4H$ stable à 20° C

Dans un tube à essais, on a ajouté 1,5 g de $C_6F_{13}C_2H_4S(C_2H_4O)_4H$, 5,0 g de $H_2O$ et 3,5 g de $C_8F_{17}CH = CH_2$. On a opéré selon le mede opératoire défini précédemment et on a obtenu une microémulsion stable à 20° C.

Microémulsions du type "eau dans huile"

Exemple 1

Préparation d'une microémulsion

$H_2O$/perfluorodécaline/$C_6F_{13}C_2H_4S(C_2H_4O)_3H$ stable à 30°C.

Dans un tube à essais, on a ajouté 3,0 g de $C_6F_{13}C_2H_4S(C_2H_4O)_3H$, 0,5 g de $H_2O$ et 6,5 g de perfluorodécaline.

On a opéré selon le mode opératoire défini précédemment et on a obtenu une microémulsion stable à 30° C.

Exemple 2

Préparation d'une microémulsion

$H_2O/C_8F_{17}CH=CH_2/C_6F_{13}(C_2H_4S)_2(C_2H_4O)_3H$ stable à 30° C.

Dans un tube à essais, on a ajouté 3,0 g de $C_6F_{13}C_2H_4S(C_2H_4O)_3H$, 0,5 g de $H_2O$ et 6,5 g de $C_8F_{17}CH=CH_2$.

On a opéré selon le mode opératoire défini précédemment et on a obtenu une microémulsion stable à 30° C.

Exemple 3

Préparation d'une microémulsion

$H_2C/C_8F_{17}CH=CH_2/C_6F_{13}C_2H_4S(C_2H_4O)_2C_2H_4S(C_2H_4O)_3H$ stable à 20° C.

Dans un tube à essais, on a ajouté 3,7 g de $C_6F_{13}C_2H_4S(C_2H_4O)_2C_2H_4S(C_2H_4O)_3$ H, 1,3 g de $H_2O$ et 5,0 g de $C_8F_{17}CH=CH_2$.

On a opéré selon le mode opératoire défini précédemment et on a obtenu une microémulsion stable à 20°C.

Exemple 4

Préparation d'une microémulsion

$H_2O$/perfluorodécaline/$C_6F_{13}C_2H_4SC_2H_4OC_2H_4$ $S(C_2H_4O)_3H$ stable à 25° C.

Dans un tube à essais, on a ajouté 2,0 g de $C_6F_{13}C_2H_4SC_2H_4OC_2H_4S(C_2H_4O)_3H$, 1,5 g de $H_2O$ et 6,5 g de perfluorodécaline.

On a opéré selon le mode opératoire défini précédemment et on a obtenu une microémulsion stable à 25° C.

Exemple 5

Préparation d'une microémulsion

$H_2O/C_8F_{17}CH=CH_2/C_6F_{13}C_2H_4S(C_2H_4O)_4H$ stable à 20° C.

Dans un tube à essais, on a ajouté 3,5 g de $C_6F_{13}C_2H_4S(C_2H_4O)_4H$, 1,0 g de $H_2O$ et 5,5 g de $C_8F_{17}CH=CH_2$.

On a opéré selon le mode opératoire défini précédemment et on a obtenu une microémulsion stable à 20° C.

Il va de soi que la présente invention n'a été décrite qu'à titre purement explicatif et nullement limitatif et que toute modification utile pourra y être apportée sans sortir de son cadre.

**Revendications**

1. Composés fluorés non ioniques répondant à la formule

$R_FC_2H_4S(R)H$ dans laquelle:

R désigne un motif $C_2H_4X$ avec X = O ou S, ou une chaîne comprenant exclusivement plusieurs de tels motifs, deux motifs consécutifs pouvant être identiques ou différents et,

$R_F$ est un groupement alkyle perfluoré à l'exclusion des composés de formule:

$R_FC_2H_4SC_2H_4OH$,

$R_FC_2H_4S(C_2H_4O)_2H$ et

$R_F(C_2H_4S)_2H$

et les mélanges de tels composés, en particulier les mélanges des produits définis ci-dessus différant entre eux par la longueur de leur chaîne perfluorée, mais dont la partie hydrophile de la molécule est identique d'un produit à l'autre.

2. Mélange de produits selon la revendication 1, caractérisé en ce que les produits du mélange ont une partie hydrophile définie.

3. Procédé de fabrication des composés selon la revendication 1, caractérisé par le fait que l'on fait réagir un composé de formule $R_FC_2H_4SH$, ($R_F$ ayant la signification donnée à cette revendication) sur une monochlorhydrine d'éthylène- ou de polyéthylène-glycol pour obtenir un premier produit, à convertir éventuellement la fonction alcool de ce produit en fonction thiol puis à faire éventuellement réagir le produit ainsi obtenu sur une monochlorhydrine d'éthylène, ou de polyéthylène- glycol, à transformer éventuellement à nouveau la fonction alcool du produit obtenu en fonction thiol et ainsi de suite jusqu'à obtention du nombre de groupements $C_2H_4O$ et/ou $C_2H_4S$ désiré.

4. Procédé de fabrication de mélanges selon la revendication 2, caractérisé en ce que l'on fait réagir un mélange de thiols de formule $R_FC_2H_4SH$ sur une monochlorhydrine d'éthylène- ou de polyéthylène- glycol pour obtenir un mélange de produits, à convertir éventuellement les fonctions alcools des produits de ce mélange en fonctions thiols puis à faire réagir éventuellement le mélange ainsi obtenu sur une monochlorhydrine d'éthylène- ou de polyéthylène- glycol, à transformer éventuellement les fonctions alcools en fonctions thiols correspondantes et ainsi de suite jusqu'à obtention du nombre de groupements $C_2H_4O$ et/ou $C_2H_4S$ désiré.

5. Application des composés selon la revendication 1 ou des mélanges selon la revendication 2 en tant qu'agents tensio-actifs non ioniques.

6. Application selon la revendication 5, caractérisée en ce que les tensio-actifs sont ajoutés à des vernis thermodurcissables.

7. Application selon la revendication 5, caractérisée en ce que les tensio-actifs sont utilisés pour former des microémulsions ou des émulsions avec des carbures fluorés ou perfluorés.

**Patentansprüche**

1. Nichtionische Fluorverbindungen, die der Formel
$R_FC_2H_4S(R)H$
entsprechen, worin:
R eine $C_2H_4X$-Gruppe mit X = O oder S oder eine Kette bezeichnet, welche ausschließlich mehrere solcher Gruppen umfaßt, wobei zwei aufeinanderfolgende Gruppen identisch oder unterschiedlich sein können, und
$R_F$ eine perfluorierte Alkylgruppe ist, unter Ausschluß von Verbindungen mit der Formel:
$R_FC_2H_4SC_2H_4OH$,
$R_FC_2H_4S(C_2H_4O)_2H$ und
$R_F(C_2H_4S)_2H$
und der Mischungen solcher Verbindungen, insbesondere der Mischungen der oben definierten Produkte, die sich untereinander durch die Länge ihrer perfluorierten Kette unterscheiden, aber deren hydrophiler Teil im Molekül von einem Produkt zum anderen identisch ist.

2. Mischung von Produkten nach Anspruch 1, dadurch gekennzeichnet, daß die Produkte der Mischung einen definierten hydrophilen Anteil besitzen.

3. Verfahren zum Herstellen von Verbindungen nach Anspruch 1, dadurch gekennzeichnet, daß man eine Verbindung mit der Formel $R_FC_2H_4SH$ (wobei $R_F$ die in diesem Anspruch gegebene Bedeutung besitzt) mit einem Monochlorhydrin von Ethylenoder Polyethylenglycol zur Umsetzung bringt, um ein erstes Produkt zu erhalten, worauf man fakultativ die Alkohol-Funktion dieses Produktes in eine Thiol-Funktion umwandelt, anschließend fakultativ das so erhaltene Produkt mit einem Monochlorhydrin von Ethylen- oder Polyethylenglycol zur Reaktion bringt, worauf man fakultativ nochmals die Alkohol-Funktion des erhaltenen Produktes in eine Thiol-Funktion umwandelt und so fortfährt, bis man die gewünschte Anzahl der $C_2H_4O$- und/oder $C_2H_4S$-Gruppen erhält.

4. Verfahren zum Herstellen von Mischungen nach Anspruch 2, dadurch gekennzeichnet, daß man eine Mischung von Thiolen mit der Formel $R_FC_2H_4SH$ mit einem Monochlorhydrin von Ethylen- oder Polyethylenglycol zur Umsetzung bringt, um eine Produktmischung zu erhalten, worauf man fakultativ die Alkohol-Funktionen der Produkte dieser Mischung in Thiol-Funktionen umwandelt, anschließend fakultativ die so erhaltene Mischung mit einem Monochlorhydrin von Ethylen- oder Polyethylenglycol zur Umsetzung bringt, worauf man fakultativ die Alkohol-Funktionen in entsprechende Thiol-Funktionen umwandelt und so fortfährt, bis man die gewünschte Anzahl der $C_2H_4O$- und/oder $C_2H_4S$-Gruppen erhält.

5. Verwendung der Verbindungen nach Anspruch 1 oder der Mischungen nach Anspruch 2 als nichtionische Tensioaktiva.

6. Verwendung nach Anspruch 5, dadurch gekennzeichnet, daß die Tensioaktiva Zusätze zu thermohärtbaren Lacken sind.

7. Verwendung nach Anspruch 5, dadurch gekennzeichnet, daß die Tensioaktiva verwendet werden, um Mikroemulsionen oder Emulsionen mit fluorierten oder perfluorierten Kohlenstoffverbindungen zu bilden.

**Claims**

1. Nonionic fluoro compounds corresponding to the formula
$R_FC_2H_4S(R)H$
in which:
R denotes a $C_2H_4X$ repeat unit with X = O or S, or a chain comprising exclusively a number of such repeat units, it being possible for two consecutive repeat units to be identical or different, and
$R_F$ is a perfluoroalkyl group, excluding the compounds of formula:
$R_FC_2H_4SC_2H_4OH$,
$R_FC_2H_4S(C_2H_4O)_2H$ and
$R_F(C_2H_4S)_2H$
and mixtures of such compounds, in particular the mixtures of the products defined above which differ from each other in the length of their perfluoro chain, but in which the hydrophilic part of the molecule is identical from one product to another.

2. Mixture of products according to Claim 1, characterized in that the products in the mixture have a specified hydrophilic part.

3. Process for the manufacture of the compounds according to Claim 1, characterized in that a compound of formula $R_FC_2H_4SH$, ($R_F$ having the meaning given in this claim) is reacted with an ethylene or polyethylene glycol monochlorohydrin to obtain a first product, for converting, if desired, the alcohol group of this product to a thiol group and then for reacting, if desired, the product thus obtained with an ethylene or polyethylene glycol monochlorohydrin, and for again converting, if desired, the alcohol group of the product obtained to a thiol group and so on until the required number of $C_2H_4O$ and/or $C_2H_4S$ groups is obtained.

4. Process for the manufacture of mixtures according to Claim 2, characterized in that a mixture of thiols of formula $R_FC_2H_4SH$ is reacted with an ethylene or polyethylene glycol monochlorohydrin to obtain a mixture of products, for converting, if desired, the alcohol groups in the products of this mixture to thiol groups and then,

if desired, for reacting the mixture thus obtained with an ethylene or polyethylene glycol monochlorohydrin, and for converting, if desired, the alcohol groups to the corresponding thiol groups and so on until the required number of $C_2H_4O$ and/or $C_2H_4S$ groups is obtained,

5. Application of the compounds according to Claim 1 or of the mixtures according to Claim 2 as nonionic surface-active agents.

6. Application according to Claim 5, characterized in that the surfactants are added to thermosetting varnishes.

7. Application according to Claim 5, characterized in that the surfactants are employed to form microemulsions or emulsions with fluoro- or perfluorocarbons.